# EUROPEAN PATENT APPLICATION

(11) **EP 1 538 139 A1**
(43) Date of publication of application: **08.06.2005**
(21) Application number: 03771336.9
(22) Date of filing: 25.07.2003
(51) Int. Cl.: C07C 5/27, C07C 7/14, C07C 13/615

(54) **PROCESS FOR THE PREPARATION OF ADAMANTANES**

(30) Priority: 29.07.2002 JP 2002219176; 30.07.2002 JP 2002220612; 30.07.2002 JP 2002220613; 30.07.2002 JP 2002220614
(71) Applicant: IDEMITSU PETROCHEMICAL CO., LTD., Tokyo 130-0015 (JP)
(72) Inventor: MASE, Jun, Shunan-shi, Yamaguchi 745-0843 (JP); MIYAMOTO, Shinji, Shunan-shi, Yamaguchi 745-0843 (JP); KOJIMA, Akio, Shunan-shi, Yamaguchi 745-0843 (JP); SAITO, Masao, Shunan-shi, Yamaguchi 745-0843 (JP)
(74) Representative: Gille Hrabal Struck Neidlein Prop Roos
(86) International application number: PCT/JP2003/009444
(87) International publication number: WO 2004/011405

(57) **Abstract**

According to the present invention, there is provided a process for producing adamantanes by isomerizing a tricyclic saturated hydrocarbon compound having 10 or more carbon atoms, comprising (A) a reaction step for isomerizing a raw material, (B) a concentration step for concentrating the adamantanes in a reaction product liquid, (C) a crystallization step for crystallizing the concentrated adamantanes, (D) a solid-liquid separation step for separating the crystallized adamantanes from slurry having precipitated crystals, (E) a washing step for washing the crystal of adamantanes obtained by the solid-liquid separation step, and (F) a drying step for drying the washed crystals of adamantanes. According to the present invention, there is provided a process for producing adamantanes by using a solid catalyst, wherein the obtained adamantanes are purified by a crystallization operation. There is provided a process for producing adamantanes by isomerizing a tricyclic saturated hydrocarbon compound, wherein the obtained adamantanes are washed by a washing solvent after separating the adamantanes by a crystallization step and a solid-liquid separation step. There is provided a process for producing adamantanes by isomerizing a tricyclic saturated hydrocarbon compound, wherein the crystal of the adamantanes which contains a liquid fraction in the range from 5 to 50% by mass is dried.

## Description

### TECHNICAL FIELD

The present invention relates to a process for producing adamantanes, and more particularly, relates to a process for producing adamantanes by using a solid catalyst which is industrially advantageous for efficiently producing high-purity adamantanes without need for troublesome operations such as waste liquid treatment while suppressing product loss as low as possible.

Also, the present invention relates to an industrially advantageous process for efficiently producing high-purity adamantanes, wherein the adamantanes produced by using a solid catalyst are purified economically without imposing environmental load.

Further, the present invention relates to an industrially advantageous process for efficiently producing high-purity adamantanes, wherein the adamantanes produced by isomerizing a tricyclic saturated hydrocarbon compound having 10 or more carbon atoms are purified economically without imposing environmental load.

### BACKGROUND ART

Adamantane is a stable compound having a high degree of symmetry with a structure composed of four cyclohexane rings in a cage-like form. Due to unique functions, adamantanes, with such adamantane structure, are known as raw materials etc. for lubricants, agrochemicals, pharmaceuticals, or high-functional industrial materials.

As a method for producing adamantanes, a method of isomerizing a tricyclic saturated hydrocarbon compound having 10 or more carbon atoms is conventionally adopted.

And, aluminum chloride is used as a catalyst in general in the isomerization reaction.

For example, adamantane is obtained by catalytically isomerizing trimethylenenorbornane (TMN) obtained by hydrogenation of dicyclopentadiene (DCPD), using aluminum chloride industrially as the catalyst.

In addition, as solid catalysts, there are disclosed those obtained by supporting active metals such as platinum, rhenium, nickel and cobalt on a cation-exchanged zeolite by an impregnation method (Japanese Patent Publication No. 2909/1977 (Show 52)).

When adamantanes are produced by using aluminum chloride as a catalyst, it is necessary to use a large amount of the catalyst, moreover the catalyst cannot be reused because the catalyst undergoes a complex formation with a heavy fraction during the reaction.

Accordingly, a large amount of waste aluminum is produced when this method is employed, and treatment of the waste causes a problem of environmental pollution.

Further, when aluminum chloride is used, since the produced adamantanes are colored, recrystallization and a decoloring step by activated carbon etc. are necessary, rendering post-treatment steps complicated.

On the other hand, in a process for producing adamantanes by using a catalyst obtained by supporting active metals such as platinum, rhenium, nickel and cobalt on a cation-exchanged zeolite by an impregnation method, the yield is low if hydrogen chloride is not present together with the catalyst (TMN conversion of 79.5%, adamantane selectivity of 10.1%, and adamantane yield of 8.0%).

Therefore, hydrogen chloride is indispensable, and due to strong corrosiveness of hydrogen chloride, it is necessary to use equipment made of expensive corrosion-resistant materials, thus causing a problem.

In addition, in the process for producing adamantanes by using the above-mentioned catalyst, although the problem caused by the use of the above-mentioned aluminum chloride catalyst can be solved, an industrial process for producing adamantanes including separation and isolation treatment of a product has not been disclosed.

In order to cope with such problems, the present inventors made extensive studies and found previously an effective process for producing adamantanes by using a solid acid supported metal catalyst without using hydrogen chloride (Japanese Patent Application No. 2000-375593).

However, in this process, although an isomerization catalyst and a method for using the catalyst at the reaction field are proposed, an industrial manufacturing process of adamantanes including separation and purification treatment of the produced adamantanes is not disclosed.

Under these circumstances, the present invention has an object of providing a process for producing adamantanes by using a solid catalyst, wherein high-purity adamantanes are efficiently produced in an industrially advantageous process in which hydrogen chloride is not used in the isomerization reaction, without need for troublesome operations such as waste liquid treatment while suppressing product loss as low as possible.

Also, an object of the present invention is to provide an industrially advantageous process for efficiently producing high-purity adamantanes, wherein adamantanes produced by using a solid catalyst are purified economically without imposing environmental load.

Further, an object of the present invention is to provide an industrially advantageous process for efficiently producing high-purity adamantanes, wherein adamantanes produced by isomerizing a tricyclic saturated hydrocarbon compound having 10 or more carbon atoms are purified economically without imposing environmental load.

### DISCLOSURE OF THE INVENTION

The present inventors made extensive studies in order to achieve the above-mentioned objects, and found that the objects can be achieved by applying certain steps to a reaction product liquid obtained by isomerizing a tricyclic saturated hydrocarbon compound having 10 or more carbon atoms.

Also, the present inventors found that the above objects can be achieved by purifying adamantanes using a crystallization operation.

Further, the present inventors found that the objects can be achieved by drying adamantanes after washing by using a washing solvent.

The present invention has been accomplished based on these findings.

That is, the present invention provides:
1. a process for producing adamantanes wherein the adamantanes are produced by isomerizing a tricyclic saturated hydrocarbon compound having 10 or more carbon atoms, comprising (A) a reaction step for isomerizing a raw material, (B) a concentration step for concentrating the adamantanes in a reaction product liquid, (C) a crystallization step for crystallizing the concentrated adamantanes, (D) a solid-liquid separation step for separating crystallized adamantanes from slurry having precipitated crystals, (E) a washing step for washing the crystal of adamantanes obtained by the solid-liquid separation step, and (F) a drying step for drying the washed crystals of adamantanes;
2. the process for producing adamantanes as set forth in 1 described above, wherein the tricyclic saturated hydrocarbon compound having 10 or more carbon atoms is a compound obtained by hydrogenation of a tricyclic unsaturated hydrocarbon compound having 10 or more carbon atoms;
3. the process for producing adamantanes as set forth in 1 described above, wherein a solid catalyst is used in the reaction step for isomerizing;
4. the process for producing adamantanes as set forth in 1 described above, wherein a flash tower or a distillation column singly or a plurality thereof in combination are used for concentration treatment in the concentration step, and at least a part of a column-top distillate is reused as a solvent in the reaction step, or at least a part of the column-top distillate is used as a recrystallization solvent in the crystallization step;
5. the process for producing adamantanes as set forth in 1 described above, wherein cooling crystallization, evaporative crystallization, or a combination thereof is used for a crystallization operation in the crystallization step;
6. the process for producing adamantanes as set forth in 1 described above, wherein the recrystallization step and a re-washing step are provided between a solid-liquid separation step or the washing step and the drying step, and at least a part of a mother liquor formed in these steps is reused for recirculation as a part of the solvent or the raw material in the reaction step, or for recirculation in the concentration step or the crystallization step;
7. the process for producing adamantanes as set forth in 1 described above, wherein the reaction step, concentration step, crystallization step, and solid-liquid separation step are operated using either a batch-wise system or a continuous system;
8. a process for producing high-purity adamantanes, wherein the adamantanes produced by isomerizing a tricyclic saturated hydrocarbon compound having 10 or more carbon atoms in the presence of a solid catalyst are purified by the crystallization operation;
9. the process for producing adamantanes as set forth in 8 described above, wherein the crystallization operation refers to a cooling crystallization operation, an evaporative crystallization operation, or a combination thereof;
10. the process for producing adamantanes as set forth in 8 described above, wherein the crystallization operation is performed using a continuous or a batch-wise system;
11. the process for producing adamantanes as set forth in 8 described above, wherein the cooling crystallization or the evaporative crystallization is performed in the temperature range from -20 to 50°C;
12. a process for producing adamantanes, wherein crude adamantanes produced by isomerizing a tricyclic saturated hydrocarbon compound having 10 or more carbon atoms are washed by a washing solvent after separating the adamantanes by a crystallization step and a solid-liquid separation step;
13. the process for producing adamantanes as set forth in 12 described above, wherein the tricyclic saturated hydrocarbon compound having 10 or more carbon atoms refers to trimethylenenorbornane;
14. the process for producing adamantanes as set forth in 12 described above, wherein the washing solvent refers to at least a solvent selected from the group consisting of alcohols, ketones and carboxylic acids having a boiling point of 150°C or less;
15. the process for producing adamantanes as set forth in 12 described above, wherein the washing solvent in the amount ranging from 10 to 300% by mass relative to the crude adamantanes is used;
16. the process for producing adamantanes as set forth in 12 described above, wherein the washing solvent in the amount ranging from 100 to 500% by mass is used to make slurry, which is then filtered;
17. a process for producing adamantanes, wherein the adamantanes are produced by isomerizing a tricyclic saturated hydrocarbon compound having 10 or more carbon atoms, and crystals of the adamantanes containing a liquid fraction in the range from 5 to 50% by mass are dried;
18. the process for producing adamantanes as set forth in 17 described above, wherein the tricyclic saturated hydrocarbon compound having 10 or more carbon atoms refers to trimethylenenorbornane;
19. the process for producing adamantanes as set forth in 17 described above, wherein the adamantanes are dried by at least a method selected from the group consisting of convective drying method, radiative drying method, and conductive drying method;
20. the process for producing adamantanes as set forth in 17 described above, wherein drying is performed by either a continuous system or a batch-wise system;
21. the process for producing adamantanes as set forth in 17 described above, wherein drying is performed under the conditions including a pressure in the range from 0.1 to 101 kPa, a temperature in the range from the boiling point of the washing solvent minus 50°C to the boiling point of the solvent; and
22. the process for producing adamantanes as set forth in 17 described above, wherein drying is performed by stirring and/or shaking.

### THE MOST PREFERRED EMBODIMENT TO CARRY OUT THE INVENTION

In the process for producing adamantanes of the present invention, each of the following processes, that is, (A) a reaction step, (B) a concentration step, (C) a crystallization step, (D) a solid-liquid separation step, (E) a washing step, and (F) a drying step, is performed.

Adamantanes in the present invention refer to a hydrocarbon compound having an adamantane structure, and include, in addition to adamantane, an alkyl-substituted adamantane having lower alkyl groups such as methyl group and ethyl group.

Next, each step of the present invention will be explained.

### (A) The Reaction Step

The reaction step refers to a step for producing adamantanes by isomerizing a raw material either by a batch-wise or a continuous system.

As the raw material described above, a tricyclic saturated hydrocarbon compound having 10 or more carbon atoms is used.

As the tricyclic saturated hydrocarbon compound having 10 or more carbon atoms, tricyclic saturated hydrocarbon compounds having 10 to 15 carbon atoms are particularly preferable, and include, for example, trimethylenenorbornane (tetrahydrodicyclopentadiene), perhydroacenaphthene, perhydrofluorene, perhydrophenalene, 1,2-cyclopentanoperhydronaphthalene, perhydoanthracene, perhydrophenanthrene, etc.

Further, alkyl-substituted compounds of these compounds, for example, 9-methylperhydroanthracene, may be cited as preferable compounds.

The tricyclic saturated hydrocarbon compounds having 10 or more carbon atoms can be easily obtained by hydrogenating tricyclic unsaturated hydrocarbon compounds having 10 or more carbon atoms such as dicyclopentadiene, acenaphthene, fluorene, phenalene, 1,2-cyclopentanonaphthalene, anthracene, phenanthrene, 9-methylanthracene or the like, by using a hydrogenation catalyst.

The hydrogenation catalyst used here is not specifically limited provided that it has hydrogenation activity, but is exemplified favorably by Raney nickel, platinum and the like.

Also, the type of the hydrogenation reactor is not specifically limited, and a so-called fixed-bed continuous reactor in which the catalyst is packed and a raw material is fed continuously, for example, is used. However, the type of the reactor is not limited by this example, and reactors of all types including conventional solid-liquid contact type and solid-gas contact type reactors in addition to continuous and batch-wise reactors can be used.

And, when a batch-wise reactor is used, reaction time is in the range from approximately 1 hour to 50 hours.

The raw material may be fed directly or together with a solvent.

In this case, the amount of the solvent for 1 part by mass of the raw material is usually in the range from 0 to 10 parts by mass, preferably in the range from 0 to 3 parts by mass, and more preferably in the range from 0.05 to 2 parts by mass.

In addition, since this hydrogenation reaction is an exothermic reaction, it is possible to minimize the amount of energy used to attain a temperature needed for the isomerization reaction by feeding resultant reaction products directly to the isomerization step.

The conditions of the hydrogenation reaction include a reaction temperature in the range usually from approximately 0 to 500°C, and preferably from 20 to 300°C, a pressure in the range usually from approximately normal pressure to 10 MPa, preferably from 3 to 6 MPa, and more preferably from 1 to 5 MPa, and the hydrogen/raw material molar ratio in the range usually from 1 or more, and preferably from 1.5 to 3.

The solid catalyst used in the isomerization reaction is not specifically limited, but is preferably a solid acid catalyst, and most preferably a solid acid supported metal catalyst.

Metals used in this solid acid supported metal catalyst include those belonging to group VIII to group X, and more specifically include iron, cobalt, nickel, ruthenium, rhodium, palladium, osmium, iridium, and platinum as preferable examples.

Among these metals, a solid acid supported platinum catalyst is particularly preferable.

Also, the solid acid which supports these metals includes various zeolites such as A-type zeolite, L-type zeolite, X-type zeolite, Y-type zeolite, and ZSM-5, etc., and metal oxides such as silica-alumina, alumina, hetero-polyacids, etc. as preferable examples.

Among these solid acids, X-type zeolite and Y-type zeolite are particularly preferable.

In addition, in view of adjusting the acid strength of carriers, catalysts carrying an alkali-earth metal and/or a rare-earth element may also be used.

And, the solid acid supported metal catalyst using the zeolite as the carrier may be produced by a process wherein at least one kind of the metals mentioned above is supported on the zeolite by using an ion-exchange method or an impregnation method.

Here, when the ion-exchange method is used, the catalyst can be obtained by contacting the zeolite with an aqueous solution of a metal salt or a metal complex to perform ion-exchanging of cation cites (H⁺, NH₄⁺, etc.), and then by calcining after drying.

Also, when the impregnation method is used, the catalyst can be obtained by mixing the zeolite with the above-mentioned aqueous solution of the metal salt or the metal complex to perform impregnation and supporting of the metals, and then by drying by evaporation using a rotary evaporator, etc.

Forms of the resultant catalyst may be either powdery or granular.

Further, the type of a reactor used in the isomerization reaction is not specifically limited, and a so-called fixed-bed continuous reactor in which the catalyst is packed and a raw material is fed continuously, for example, is used. However, the type of the reactor is not limited to this example, and reactors of all types including conventional solid-liquid contact type and solid-gas contact type reactors in addition to continuous and batch-wise reactors can be used.

In the isomerization reaction, the hydrogenated compound described above may be used in the reaction after purification or directly without purification.

In either case, the reaction may be performed in the presence of a solvent.

In this case, the amount of the solvent for 1 part by mass of the raw material is usually in the range from approximately 0 to 10 parts by mass, preferably in the range from approximately 0 to 3 parts by mass, and more preferably in the range from 0.05 to 2 parts by mass.

When the hydrogenated compound is used directly without purification, the above-mentioned amount can be adjusted by either removing a part of the solvent used in the hydrogenation step or adding the solvent additionally.

The conditions of the isomerization reaction include a reaction temperature in the range usually from approximately 150 to 500°C, and preferably from 200 to 400°C, a pressure in the range usually from approximately normal pressure to 20 MPa, preferably from 2 to 8 MPa. The reaction may be preferably performed in the presence of hydrogen from the viewpoint of yield increase.

Further, in the present invention, the isomerization of the above-mentioned tricyclic saturated hydrocarbon compound having 10 or more carbon atoms can be performed in the presence of a monocyclic saturated hydrocarbon compound, an aromatic compound, water and/or alcohols.

Here, the concurrently present monocyclic saturated hydrocarbon compound includes, for example, cyclopentane, cyclohexane, ethylcyclohexane, methylcyclohexane and the like.

Cyclohexane, ethylcyclohexane, or a mixture thereof is particularly preferable.

Also, the aromatic compound includes, for example, aromatic hydrocarbon compounds such as benzene, toluene, xylenes, naphthalene, anthracene, etc.; oxygenated aromatic compounds such as phenol, benzaldehyde, benzoic acid, benzyl alcohol, anisole, etc.; aromatic compounds containing nitrogen such as aniline, nitrobenzene, etc.; and halogenated aromatic compounds such as chlorobenzene, bromobenzene, etc.

Among these aromatic compounds, aromatic hydrocarbon compounds such as benzene, toluene, xylenes, naphthalene, anthracene, etc. are more preferable, and benzene is particularly preferable.

On the other hand, alcohols include, for example, monovalent alcohols such as methyl alcohol, isopropyl alcohol, tert-butyl alcohol, benzyl alcohol, etc., and polyvalent alcohols such as ethylene glycol, glycerin, etc.

The amount of these concurrently present compounds is not specifically limited, and may be selected depending on various situations.

### (B) The Concentration Step

The concentration step refers to a step for concentrating a product liquid of the isomerization reaction obtained in the above-mentioned isomerization step to a concentration at which efficient crystallization can be performed in the next crystallization step, wherein the product liquid is concentrated with either a batch-wise system or a continuous system by using a flash tower or a distillation column singly or a plurality thereof in combination, and the solvent and low-boiling byproducts (impurities) are removed.

In the concentration step, usually light gases such as unreacted hydrogen are removed using a flash tower, and one distillation column is used to complete the concentration.

And, the concentration is performed to obtain the concentration of adamantanes in the range usually from 10 to 50% by mass, preferably from 10 to 45% by mass, more preferably from 10 to 40% by mass, furthermore preferably from 20 to 45% by mass, and most preferably from 20 to 40% by mass.

If the degree of concentration is too low, the recovery yield of the adamantanes in the crystallization step is poor, while if the degree of concentration is too high, the impurities are also concentrated proportionately and easily incorporated into the adamantanes.

Light distillate coming out of the column top can be used in part or as a whole as a solvent in the isomerization step or in the hydrogenation step depending on the case, thereby the use of a fresh reaction solvent can be minimized, and the raw material and reaction intermediate distillate, or adamantanes contained in the light distillate fraction can be used by recirculation.

Also, by using a part or the whole of the light distillate fraction in the next crystallization step as a recrystallization solvent, the use of a fresh recrystallization solvent can be minimized.

The amount of light distillate which is recirculated into the reaction step or used as a recrystallization solvent depends on the concentration of adamantanes and the concentration of other impurities, or on the flow rate balance in the reaction process, and the product having a desired purity can be obtained efficiently by adjusting the amount properly.

### (C) The Crystallization Step

The crystallization step refers to a step for crystallizing the adamantanes from the concentrate obtained in the above-mentioned concentration step using a batch-wise system or a continuous system.

The crystallization step can be performed by using conventional cooling crystallization, evaporative crystallization or the combination thereof.

The operating temperature in the crystallization step depends on the concentration of the adamantanes in the above-mentioned concentrate.

In the case of continuous crystallization, the temperature is in the range usually from -20 to 50°C, preferably from 0 to 40°C, and more preferably from 0 to 30°C.

When the temperature is lower than -20°C, a large amount of energy is consumed in cooling, whereas when the temperature is higher than 50°C, the solubility of the adamantanes in the solvent becomes large, rendering the recovery efficiency of the adamantanes low.

In addition, for a similar reason, in any of other crystallization procedures, it is advantageous to set the final temperature in the crystallization step at the temperature at which the solubility of the adamantanes is in the range from approximately 0.5 to 25% by mass, and preferably in the range from 5 to 15% by mass.

When impurities that may cause problems in the product quality are contained after single crystallization, it is possible to perform recrystallization immediately after crystallization, or it is possible to repeat a plurality of times the recrystallization, solid-liquid separation step and washing steps after performing post-steps of solid-liquid separation and washing.

### (D) The Solid-Liquid Separation Step

The solid-liquid separation step refers to a step for separating the adamantanes crystallized in the above-mentioned crystallization step from the solvent by a batch-wise system or a continuous system.

The solid-liquid separation step may be performed by a conventional method using a filter cloth, a sintered metal, or the like.

In this case, a part of the separated mother liquor is usually exhausted to the outside of the system in order to reduce the concentration of impurities.

And, by recirculating a part or the whole of the residual liquid into the concentration step and the crystallization step, the loss of the adamantanes can be reduced to a minimum, or by using it as a part of the solvent in the isomerization step or in the hydrogenation step depending on the case, or as a part of the raw material, addition of a fresh solvent can be eliminated, and also the raw material and the reaction intermediate distillate, or the adamantanes contained in the light distillate can be used by recirculation.

The degree of solid-liquid separation is such that the liquid content in the separated crystal cake is usually 50% by mass or less, and is preferably in the range from 5 to 30% by mass.

The amount of mother liquor that is exhausted to the outside of the system or is recirculated depends on the concentration of the adamantanes and other impurities, or on the flow rate balance in the reaction process, and the product having a desired purity can be obtained efficiently by adjusting the amount properly.

### (E) The Washing Step

The washing step refers to a step for removing by washing the solvent that was not fully removed in the above-mentioned solid-liquid separation, with a washing solvent.

Almost all the organic solvents may be used as the washing solvent, but polar solvents having low solubility of the adamantanes such as alcohols, ketones, carboxylic acids and the like are used preferably in order not to reduce the recovery yield of the adamantanes.

Also, when the washed adamantanes are treated directly in the next drying step, solvents having low boiling points are preferable, and usually those having the boiling point of 150°C or lower are used preferably.

Examples of these solvents include methanol, ethanol, propanol, isopropanol, acetone, methylethylketone, diethylketone, acetic acid. carbon tetrachloride, and the like.

The operating temperature of washing is usually in the range from room temperature to the boiling point of the washing solvent, and is preferably in the range from -20 to 50°C.

### (F) The Drying Step

The drying step refers to a step for drying the crystal of the washed adamantanes obtained in the above-mentioned washing step.

The drying step may be performed by using an industrial, conventional dryer such as reduced pressure type, heating type, etc.

In addition, a drying procedure may be either with a continuous system or a batch-wise system.

Since the purpose of the drying treatment is removal of the washing solvent, operating conditions are different depending on the kind of the solvent used in the above-mentioned washing step. The conditions include a normal pressure of (101 kPa) or less and preferably in the range from 5 to 101 kPa, and a temperature usually at the boiling point of the solvent or less, and preferably in the range from 20 to 60°C.

As described above, it is possible to efficiently produce high-purity adamantanes without needing troublesome operations such as waste liquid treatment, etc., while minimizing product loss.

In the following, purification methods of the adamantanes are explained in terms of operations including crystallization, washing and drying according to the present invention.

The present invention refers to a process for producing high-purity adamantanes, wherein a tricyclic saturated hydrocarbon compound having 10 or more carbon atoms is isomerized in the presence of a solid catalyst, a resultant reaction product liquid is used as a raw material for crystallization, and the adamantanes are purified by crystallization operation.

In the present invention, the concentration of the adamantanes in the raw material for crystallization is in the range approximately from 10 to 45% by mass, preferably in the range from 10 to 40% by mass, more preferably in the range from 20 to 45% by mass, and furthermore preferably in the range from 20 to 40% by mass. The temperature is not specifically limited except that at the temperature or higher all the adamantanes are dissolved.

In the case where the reaction product liquid having a concentration of the adamantanes below 10% by mass is used as the raw material for crystallization, it is advantageous to concentrate the product liquid by distillation, etc. beforehand.

This is because, if the concentration of adamantanes is too low, the recovery yield of the adamantanes in the crystallization step becomes low.

On the other hand, if the concentration of adamantanes is too high, operation becomes difficult because the viscosity of slurry increases during crystallization.

In the present invention, either of cooling crystallization or evaporative crystallization may be used in the crystallization operation, or also both of them may be combined.

This crystallization operation may be performed using either of a continuous system or a batch-wise system.

In the case where a continuous system is used in the cooling crystallization, the operation temperature is usually in the range from -20 to 50°C, preferably in the range from 0 to 40°C, and more preferably in the range from 0 to 30°C.

When the temperature is lower than -20°C, a large amount of energy is consumed in the cooling, whereas when the temperature is higher than 50°C, the solubility of the adamantanes in the solvent becomes large, rendering the recovery efficiency of the adamantanes low.

In the case where a batch-wise system, for a similar reason, it is advantageous to control the final temperature to preferably in the range from -20 to 50°C, and more preferably in the range from 0 to 30°C.

In addition, for a similar reason, in any of other crystallization procedures, it is advantageous to set the final temperature in the crystallization step at the temperature at which the solubility of the adamantanes is in the range from 0.5 to 25% by mass, and preferably in the range from 5 to 15% by mass.

The solid-liquid separation of the crystallization liquid containing the crystallized adamantanes is performed by a conventional method using a filter cloth, a sintered metal, or the like.

In addition, when adamantanes having purity higher than the desired one cannot be obtained in single crystallization, recrystallization from the obtained crystal may be performed by dissolving the crystal in a usual organic solvent.

When the crystal is dissolved in the solvent, solvents having low solubility for the adamantanes are not desirable.

Such crystallization solvents, that is, inappropriate solvents, include alcohols, ketones, carboxylic acids and the like.

The present invention refers to a process for producing adamantanes by isomerizing a tricyclic saturated hydrocarbon compound having 10 or more carbon atoms, wherein the resultant adamantanes are separated by crystallization and solid-liquid separation steps, followed by washing by using a washing solvent.

That is, in the present invention, a wet cake (crude adamantanes) obtained by removing a liquid containing an unreacted raw material of TMN, byproducts etc. is washed in the washing step after solid-liquid separation.

Here, the byproducts refer to hydrocarbons having 10 or more carbon atoms.

Since the unreacted raw material of TMN, the byproducts etc. are contained in the wet cake, liquid removal is performed in the solid-liquid separation step until the liquid content reaches 5 to 50% by mass for industrial use.

If the liquid content is high, the washing efficiency of the wet cake becomes low, and if it is low, the liquid removal requires longer time and a large amount of energy.

Operations in the washing step include a displacement washing procedure in which the solvent is penetrated into the wet cake, or a filtration procedure in which the solvent is added to make slurry of the wet cake, or the like.

As a washing solvent, at least one polar solvent selected from the group consisting of alcohols, ketones, and carboxylic acids having a boiling point of 150°C or less may be used.

For example, methanol, ethanol, 1-propanol, isopropyl alcohol, acetone, methylethylketone, diethylketone, acetic acid, carbontetrachloride and the like are included.

The operating temperature of washing is usually in the range from room temperature to the boiling point of the washing solvent, preferably in the range from -20 to 50°C, more preferably in the range from 0 to 40°C, and furthermore preferably in the range from 0 to 30°C.

When the displacement washing is performed, the amount of the washing solvent with respect to the wet cake is usually in the range from 10 to 300% by mass, and preferably in the range from 20 to 100% by mass.

If the amount of the used solvent is large, the recovery yield of the adamantanes becomes low, because they are dissolved in the washing solvent.

If the amount of the used solvent is small, the purity of the adamantanes becomes low because of poor washing efficiency.

When slurry is made by adding the washing solvent, the amount of the washing solvent with respect to the wet cake is in the range from 100 to 500% by mass, and preferably in the range from 150 to 400% by mass.

If the amount of the washing solvent is large, the recovery efficiency becomes low because the adamantanes are dissolved in the washing solvent.

By the washing operation of the present invention, substantial part of the unreacted raw material of TMN, and the byproducts can be eliminated.

For crystals of the adamantanes obtained by the washing operation, by performing operations including evaporation of the adhered washing solvent, drying etc., crystals of high-purity adamantanes can be obtained.

The present invention refers to a process for producing adamantanes by isomerizing a tricyclic saturated hydrocarbon compound having 10 or more carbon atoms, wherein the resultant adamantanes are separated by a washing step and are dried.

The washed crystals of adamantanes contain 5 to 50% by mass of the washing solvent etc. as the liquid content.

The washing solvent contained in the crystal of adamantanes includes, as described above, at least one kind of the polar solvents selected from the group consisting of alcohols, ketones, and carboxylic acids having a boiling point of 150°C or less.

If the boiling point of the washing solvent is higher than 150°C, the adamantanes is lost by sublimation etc. during drying, giving rise to large product loss.

The drying temperature is less than or equal to the boiling point of the washing solvent, preferably in the range from the boiling point of the solvent minus 50°C to the boiling point of the solvent, and more preferably in the range from 20 to 60°C.

The drying pressure is less than or equal to normal pressure (101 kPa), preferably in the range from 0.1 to 101 kPa, and more preferably in the range from 5 to 100 kPa.

Drying can be performed by using at least one method selected from the methods consisting of convective (hot air) drying, radiative drying, conductive drying, and vacuum drying.

Specifically, drying can be performed in a stream of a non-reactive gas, under a reduced pressure, or with infrared irradiation, or by heat conduction through a jacket, or the like.

As the non-reactive gas, nitrogen, argon etc. may be included.

Also, drying may be performed by a continuous system or a batch-wise system.

Further, it is advantageous to perform the drying while stirring and shaking of the adamantanes in the drying step.

When the drying is performed without stirring or shaking, the crystal of adamantanes may solidify, which may cause difficulty in handling.

By performing the drying as described above, the content of the liquid such as the washing solvent etc. in the crystal of adamantanes decreases usually to the range from 0 to 1% by mass.

Next, the present invention will be described in more detail with reference to examples, which however shall never limit the present invention thereto.

### Preparation Example 1: Preparation of a solid acid supported metal catalyst

In 2,000 g of pure water, 235 g of sodium ion-exchanged Y-type zeolite was suspended by stirring, to which an aqueous solution of dilute nitric acid was added to adjust the pH of the suspended slurry to 5.5.

Next, an aqueous solution prepared by dissolving 246 g of lanthanum nitrate hexahydrate in 500 g of warm water was added slowly to the above-mentioned suspended slurry.

Subsequently, the slurry was warmed to 90°C and was stirred for 30 minutes, and was then filtered and washed. Next, the filtered and washed cake was dried overnight at 110°C, and further calcined at 600°C for 3 hours.

The calcined powder was again added to 2,000 g of pure water to prepare suspended slurry, to which 228 g of ammonium sulfate was added. The resultant slurry was stirred at 95°C for 30 minutes, and then was filtered and washed.

The washed cake was again suspended in 2,000 g of pure water, and the similar ion-exchange operation was performed twice successively.

Then, after drying overnight at 110°C, the cake was put into a tubular container, and was steamed at 510°C for 30 minutes in the presence of a 100% steam.

Next, the resultant powder was suspended in 2,000 g of pure water, to which 32 g of sulfuric acid having 25% by mass was added slowly, followed by heating at 95°C for 30 minutes.

Subsequently, after performing filtering and washing, the resultant material was again suspended in 2,000 g of pure water, to which 180 g of an aqueous solution of tetraammineplatinum chloride of 1.71% by mass was added, followed by stirring at 60°C for 30 minutes.

After filtering and washing, the obtained material was dried overnight at 110°C to yield a catalyst composed of Y-type zeolite containing lanthanum and having 0.87% by mass of platinum supported by ion-exchange.

### Example 1

### (1) The reaction and concentration steps

In a stainless-steel reaction tube, 20 g of the catalyst prepared in Preparation Example 1 was packed and was calcined in air stream at 300°C for 3 hours.

After displacing by nitrogen, the catalyst was hydrogenated in a stream of hydrogen at 300°C for 2 hours under normal pressure.

Subsequently, feeding of a mixture solution of decalin and trimethylenenorbornane (TMN) in a mass ratio of 2:1 and hydrogen was started, and isomerization reaction was continuously performed under the conditions including a temperature of 300°C, a pressure of 5 MPa, a WHSV of 2.4 h⁻¹, and a hydrogen/TMN molar ratio of 2.

The adamantane concentration in the reaction product liquid was 7% by mass.

The reaction product liquid of 1,000 g was concentrated by using a 15-tray distillation column at normal pressure until the adamantane concentration reached 26% by mass.

In the concentrated liquid, 15% by mass of unreacted TMN and 59% by mass of other impurities were contained.

### (2) The purification step

The concentrated liquid obtained in (1) mentioned above was used as a crystallization raw material, and 180 g of it was put into a flask and dissolved at 120°C by stirring.

By continuously stirring, the liquid was cooled slowly down to 10°C to render crystallization, and slurry with crystallized adamantane was obtained.

Next, the slurry was filtered by using a glass filter having a 70 µm pore size, and 60 g of the resultant filtered cake was washed by using 35 g of isopropanol.

Into an egg-plant type flask, 33 g of the crystal obtained after washing was added, and was dried by using an evaporator at 50°C under a pressure of 25 kPa for 30 minutes with stirring.

The crystal of 28 g obtained after drying was analyzed by gas chromatography, from which the purity was found to be 98% by mass.

Impurities included 0.1% by mass of isopropanol, and 2% by mass in total of TMN and other impurities.

The yield of adamantane with respect to the raw material of TMN was 8% by mass.

### Example 2

### (1) The reaction and concentration steps

The isomerization reaction was performed under the conditions similar to those used in Example (1) except for the point that in place of decalin used in (1) in Example 1, a solution prepared by mixing the column-top distillate and the crystallization mother liquor obtained during the concentration time of the reaction product liquid in a ratio of 3:1 by mass was used.

The adamantane concentration in the reaction product liquid was 8% by mass.

The reaction product liquid of 1,000 g was concentrated by using a 15-tray distillation column at normal pressure until the adamantane concentration reached 28% by mass.

In the concentrated liquid, 17% by mass of unreacted TMN and 55% by mass of other impurities were contained.

### (2) The purification step

Crystallization, washing, and drying were performed in a manner similar to (2) of Example 1.

The crystal of 33 g obtained after drying was recovered and analyzed by gas chromatography, from which the purity was found to be 98% by mass.

Impurities included 0.1% by mass of isopropanol, TMN and other impurities in total 2% by mass.

The adamantane yield with respect to the raw material of TMN was 14% by mass.

### Example 3

### (1) The reaction and concentration steps

In a stainless-steel reaction tube, 20 g of the catalyst prepared in Preparation Example 1 was packed and was calcined in air stream at 300°C for 3 hours.

After displacing by nitrogen, the catalyst was hydrogenated in a stream of hydrogen at 300°C for 2 hours under normal pressure.

Thereafter, feeding of a solution of trimethylenenorbornane (TMN) and hydrogen was started, and isomerization reaction was continuously performed under the conditions including a temperature of 300°C, a pressure of 5 MPa, a WHSV of 2.4 h⁻¹, and a hydrogen/TMN molar ratio of 2.

The reaction product liquid was concentrated by using a 15-tray distillation column at normal pressure at a column-bottom temperature of 180°C until the adamantane concentration reached 30% by mass.

### (2) The purification step

The concentrated liquid obtained in (1) mentioned above was used as a crystallization raw material, and 300 g of it was put into a flask and dissolved at 120°C by stirring.

By continuously stirring, the liquid was cooled down to 10°C to render crystallization, and slurry with crystallized adamantane was obtained. Next, the slurry was filtered by using a glass filter having a 70 µm pore size, and crude adamantane crystal was obtained.

The crude adamantane crystal was analyzed by gas chromatography, from which the purity of the crude adamantane crystal was found to be 80% by mass. Impurities included 15% by mass ofunreacted trimethylenenorbornane (TMN) and 5% by mass of byproducts.

To 75 g of the crude adamantane crystal placed on a filter having a 70 µm pore size, 75 g of isopropyl alcohol was added to perform displacement washing by suction filtration.

The resultant adamantance crystal was air-dried to evaporate isopropyl alcohol, and 59 g of adamantane crystal was obtained.

The adamantane crystal was analyzed by gas chromatography. The purity of the adamantane crystal was found to be 98% by mass, and impurities included 1% by mass of unreacted TMN and 1% by mass of by products.

### Comparative Example 1

Reaction, concentration, and crystallization steps were performed in a manner similar to Example 3, and slurry with crystallized adamantane was obtained. The slurry was filtered by using a glass filter having a 70 µm pore size to obtain crude adamantane crystal.

The crude adamantane crystal was air-dried without washing by isopropyl alcohol to obtain 70 g of adamantane crystal.

The crystal was analyzed by gas chromatography. The purity of adamantane crystal was found to be 87% by mass, and impurities included 9% by mass of unreacted TMN and 4% by mass of byproducts.

### Example 4

### (1) The reaction and concentration steps

In a stainless-steel reaction tube, 20 g of the catalyst prepared in Preparation Example 1 was packed and was calcined in air stream at 300°C for 3 hours.

After displacing by nitrogen, the catalyst was hydrogenated in a stream of hydrogen at 300°C for 2 hours under normal pressure.

Subsequently, feeding of a solution of trimethylenenorbornane (TMN) and hydrogen was started, and isomerization reaction was continuously performed under the conditions including a temperature of 300°C, a pressure of 5 MPa, a WHSV of 2.4 h⁻¹, and a hydrogen/TMN molar ratio of 2.

The reaction product liquid was concentrated by using a 15-tray distillation column at normal pressure at a column-bottom temperature of 180°C until the adamantane concentration reached 30% by mass.

### (2) The purification step

The concentrated liquid obtained in (1) mentioned above was used as a crystallization raw material, and 300 g of it was put into a flask and dissolved at 120°C by stirring.

By continuously stirring, the liquid was cooled down to 10°C to render crystallization, and slurry with crystallized adamantane was obtained.

Next, the slurry was filtered by using a glass filter having a 70 µm pore size, and crude adamantane crystal was obtained.

The crude adamantane crystal was analyzed by gas chromatography, from which the purity of crude adamantane crystal was found to be 80% by mass. Impurities included 15% by mass of unreacted TMN and 5% by mass of byproducts.

To 75 g of the crude adamantane crystal placed on a filter having a 70 µm pore size, 75 g of isopropyl alcohol was added to perform displacement washing by suction filtration.

From the results of analysis of the resultant adamantane crystal, the purity of the adamantane crystal was found to be 83% by mass, and impurities included 15% by mass of isopropyl alcohol and 2% by mass in total ofunreacted TMN and byproducts.

Into an egg-plant type flask, 50 g of the crystal obtained after washing was added, and was dried at 50°C under a pressure of 25 kPa for 30 minutes with stirring and rotating. After bringing the flask to room temperature and to normal pressure, 42 g of lump-free and powdery crystal was obtained.

The adamantane crystal after the drying treatment was analyzed. As a result, the purity of the adamantane crystal was found to be 98% by mass, and impurities included 0.1% by mass of isopropyl alcohol and 2% by mass in total ofunreacted TMN and byproducts.

The recovery yield of the adamantane was 99% by mass.

### INDUSTRIAL APPLICABILITY

According to the present invention, a process for producing adamantanes by using a solid catalyst is provided, wherein high-purity adamantanes are produced efficiently without using hydrochloric acid in isomerization reaction and also without the need for troublesome operations such as waste liquid treatment while suppressing product loss as low as possible in an industrially advantageous process.

According to the process of the present invention, high-purity adamantanes are efficiently produced in an industrially advantageous process, wherein the adamantanes obtained by using a solid catalyst are purified economically by crystallization treatment and without imposing environmental load.

According to the process of the present invention, high-purity adamantanes are efficiently produced in an industrially advantageous process, wherein the adamantanes obtained by isomerizing a tricyclic saturated hydrocarbon compound are purified economically by washing treatment and without imposing environmental load.

## Claims

1. A process for producing adamantanes by isomerizing a tricyclic saturated hydrocarbon compound having 10 or more carbon atoms, wherein the process comprises: (A) a reaction step for isomerizing a raw material; (B) a concentration step for concentrating the adamantanes in a reaction product liquid; (C) a crystallization step for crystallizing the concentrated adamantanes; (D) a solid-liquid separation step for separating crystallized adamantanes from slurry having precipitated crystals; (E) a washing step for washing the crystal of adamantanes obtained by the solid-liquid separation step; and (F) a drying step for drying the washed crystals of adamantanes.

2. The process for producing adamantanes according to Claim 1, wherein the tricyclic saturated hydrocarbon compound having 10 or more carbon atoms is a compound obtained by hydrogenation of a tricyclic unsaturated hydrocarbon compound having 10 or more carbon atoms.

3. The process for producing adamantanes according to Claim 1, wherein a solid catalyst is used in the reaction step for isomerizing.

4. The process for producing adamantanes according to Claim 1, wherein a flash tower or a distillation column singly or a plurality thereof in combination are used for concentration treatment in the concentration step, and at least a part of a column-top distillate is reused as a solvent in the reaction step, or at least a part of the column-top distillate is used as a recrystallization solvent in the crystallization step.

5. The process for producing adamantanes according to Claim 1, wherein cooling crystallization, evaporative crystallization, or the combination thereof are used for the crystallization operation in the crystallization step.

6. The process for producing adamantanes according to Claim 1, wherein a recrystallization step and a re-washing step are provided between the solid-liquid separation step or the washing step and the drying step, and at least a part of a mother liquor formed in these steps is reused by recirculating as a part of the solvent or the raw material in the reaction step, or by recirculating to the concentration step or to the crystallization step.

7. The process for producing adamantanes according to Claim 1, wherein the reaction step, concentration step, crystallization step, and solid-liquid separation step are operated using either a batch-wise system or a continuous system.

8. A process for producing adamantanes, wherein the adamantanes produced by isomerizing a tricyclic saturated hydrocarbon compound having 10 or more carbon atoms in the presence of a solid catalyst are purified by a crystallization operation.

9. The process for producing adamantanes according to Claim 8, wherein the crystallization operation refers to a cooling crystallization operation, a evaporative crystallization operation, or the combination thereof.

10. The process for producing adamantanes according to Claim 8, wherein the crystallization operation is performed using a continuous system or a batch-wise system.

11. The process for producing adamantanes according to Claim 8, wherein the cooling crystallization operation or the evaporative crystallization operation is performed in the temperature range from -20 to 50°C.

12. A process for producing adamantanes, wherein crude adamantanes produced by isomerizing a tricyclic saturated hydrocarbon compound having 10 or more carbon atoms are washed by a washing solvent after separating the adamantanes by a crystallization step and a solid-liquid separation step.

13. The process for producing adamantanes according to Claim 12, wherein the tricyclic saturated hydrocarbon compound having 10 or more carbon atoms refers to trimethylenenorbornane.

14. The process for producing adamantanes according to Claim 12, wherein the washing solvent refers to at least a solvent selected from the group consisting of alcohols, ketones and carboxylic acids having a boiling point of 150°C or less.

15. The process for producing adamantanes according to Claim 12, wherein the washing solvent in the amount ranging from 10 to 300% by mass relative to the crude adamantanes is used.

16. The process for producing adamantanes according to Claim 12, wherein the washing solvent in the amount ranging from 100 to 500% by mass is used to make slurry, which is then filtered.

17. A process for producing adamantanes, wherein the adamantanes are produced by isomerizing a tricyclic saturated hydrocarbon compound having 10 or more carbon atoms, wherein crystals of the adamantanes containing a liquid fraction in the range from 5 to 50% by mass are dried.

18. The process for producing adamantanes according to Claim 17, wherein the tricyclic saturated hydrocarbon compound having 10 or more carbon atoms refers to trimethylenenorbornane.

19. The process for producing adamantanes according to Claim 17, wherein the adamantanes are dried by at least a method selected from the group consisting of convective drying method, radiative drying method, and conductive drying method.

20. The process for producing adamantanes according to Claim 17, wherein drying is performed by either a continuous system or a batch-wise system.

21. The process for producing adamantanes according to Claim 17, wherein the drying is performed under the conditions including a pressure in the range from 0.1 to 101 kPa, a temperature in the range from the boiling point of the washing solvent minus 50°C to the boiling point of the solvent.

22. The process for producing adamantanes according to Claim 17, wherein the drying is performed by stirring and/or shaking.
